# EUROPEAN PATENT SPECIFICATION

(11) **EP 3 848 354 B1**
(45) Date of publication and mention of the grant of the patent: **27.07.2022**
(21) Application number: 21158883.5
(22) Date of filing: 09.09.2013
(51) Int. Cl.: C07D 215/26, A61K 31/4704, A61P 11/06, A61P 11/08

(54) **PROCESS FOR THE PREPARATION OF INDACATEROL AND INTERMEDIATES THEREOF**
VERFAHREN ZUR HERSTELLUNG VON INDACATEROL UND ZWISCHENPRODUKTE DAVON
PROCÉDÉ DE PRÉPARATION D'INDACATÉROL ET INTERMÉDIAIRES DE CELLE-CI

(30) Priority: 21.09.2012 WO PCT/EP2012/003961
(43) Date of publication of application: 14.07.2021
(62) Divisional of application: 16189965.3
(73) Proprietor: Crystal Pharma, S.A.U., 47151 Boecillo Valladolid (ES)
(72) Inventor: Antonio, Lorente Bonde-Larsen, 47151 Boecillo - Valladolid (ES); Yolanda, Fernández Sainz, 47151 Boecillo - Valladolid (ES); Jesús, Iglesias Retuerto, 47151 Boecillo - Valladolid (ES); Javier, Gallo Nieto, 47151 Boecillo - Valladolid (ES)
(74) Representative: Plougmann Vingtoft a/s

(56) References cited:
- WO-A1-00/75114
- WO-A1-2004/076422
- WO-A1-2007/124898
- WO-A2-2004/016578

## Description

### Field of the invention.

The present invention relates to new and improved processes for the preparation of Indacaterol and pharmaceutically acceptable salts thereof as well as intermediates for the preparation of Indacaterol.

### Background of the invention

The compound 5-[(R)-2-(5,6-diethyl-indan-2-ylamino)-1-hydroxyethyl]-8-hydroxy-(1H)-quinolin-2-one, which is known as Indacaterol (INN), and its corresponding salts are beta-selective adrenoceptor agonists with a potent bronchodilating activity. Indacaterol is especially useful for the treatment of asthma and chronic obstructive pulmonary disease (COPD) and is sold commercially as the maleate salt.

WO 00/75114 and WO 2004/076422 describe the preparation of Indacaterol for the first time through the process:

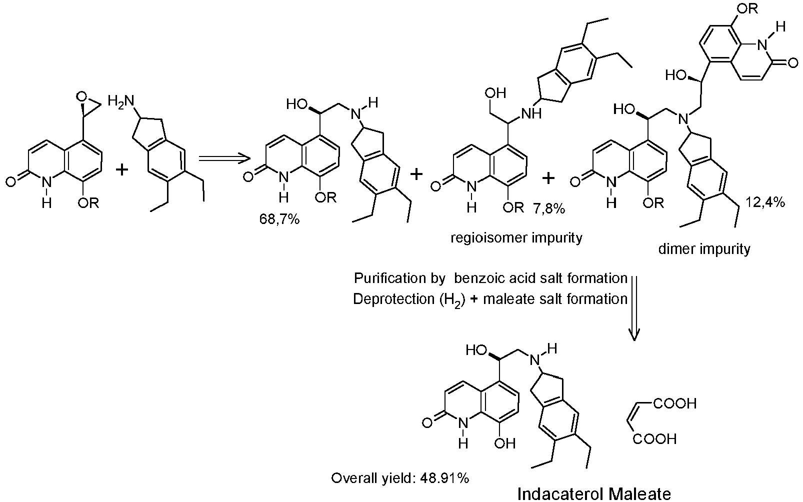

The condensation between the indanolamine and the quinolone epoxide leads to the desired product but always with the presence of a significant amount of impurities, the most significant being the dimer impurity, which is the consequence of a second addition of the product initially obtained with another quinolone epoxide, as well as the formation of another isomer which is the result of the addition of the indanolamine to the secondary carbon of the epoxide.

In addition, the reaction conditions to achieve the opening of the epoxide require high energies (ex. 21 of WO 00/75114) with temperatures of 110 °C or more for several hours, which favours the appearance of impurities.

WO 2004/076422 discloses the purification of the reaction mixture by the initial formation of a salt with an acid, such as tartaric acid or benzoic acid, hydrogenation and final formation of the maleate salt. However, the yield achieved by the end of the process is only 49% overall.

It has been found that impurities of tartrate and benzoate salts can exist in the final product as a result of displacing the tartrate or benzoate with maleate without prior neutralization to Indacaterol base. In addition, WO 2004/076422 discloses that proceeding via the free base of Indacaterol is not viable due to its instability in organic solvents. WO 00/75114 does disclose a method proceeding via the Indacaterol free base, but it is not isolated in solid form.

WO 2004/076422 furthermore discloses the method for obtaining the quinolone epoxide from the corresponding α-haloacetyl compound by reduction in the presence of a chiral catalyst, such as an oxazaborolidine compound, by proceeding via the α-halohydroxy compound.

Documents WO 2007/124898 and WO 2004/013578 disclose 8-(benzyloxy)-5-[(1R)-2-bromo-1-{[tert-butyl(dimethyl)silyl]oxy}ethyl]quinolin-2(1H)-one and 8-(benzyloxy)-5-[(1R)-2-bromo-1-{tetrahydro-2H-pyran-2-yl-oxy}ethyl]quinolin-2(1H)-one, respectively. Said documents are however not concerned with the preparation of Indacaterol.

Documents WO 2004/016578 and WO 2007/124898 disclose ethers suitable for treating respiratory diseases and diseases associated with β2 adrenergic receptor activity, respectively.

There exists, therefore, the need to develop an improved process for obtaining Indacaterol and salts thereof, which overcomes some or all of the problems associated with known methods from the state of the art. More particularly, there exists the need for a process for obtaining Indacaterol and pharmaceutically acceptable salts thereof, which results in a higher yield and/or having fewer impurities in the form of the dimer and regioisomers impurities and/or salts other than the desired pharmaceutically acceptable salt.

### Summary of the invention

In one aspect of the invention, it concerns a process for preparing Indacaterol or a pharmaceutically acceptable salt thereof comprising reacting the compound of formula I with 2-amino-5,6-diethylindan of formula II, preferably in the presence of a base, to the compound of formula III and then converting the compound of formula III to Indacaterol or a pharmaceutically acceptable salt thereof: 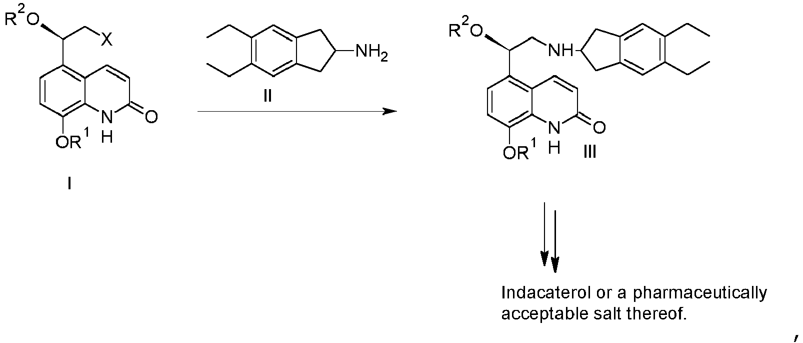 wherein R¹ is benzyl, R² is a protecting group, which is stable under mildly alkaline conditions, and X is a halogen selected from the group consisting of chloro, bromo, and iodo.

This process avoids the formation of the dimers and regiostereoisomers associated with the processes known in the art, e.g. in WO 2004/076422, since it avoids the use of the epoxy compound used in the prior art processes. This facilitates the purification of the compound of formula III, possible subsequent intermediates in the process, as well as the final product. The process of the invention furthermore has gentler reaction conditions than the processes known in the art and results in a yield of more than 70% and in some cases more than 80%.

R¹ is a protecting group commonly known in the art for protecting phenol groups. R² is a protecting group, which is stable under mildly alkaline conditions.

A further aspect of the invention concerns a process for the preparation of the compound of formula III or a salt thereof by reacting the compound of formula I with 2-amino-5,6-diethylindan of formula II to the compound of formula III. Optionally, the compound of formula III is converted to a salt thereof by addition of an acid.

In another aspect of the invention, it concerns a process for the preparation of a pharmaceutically acceptable salt of Indacaterol by obtaining Indacaterol, isolating it in solid form, and reacting it with a suitable acid, such as maleic acid.

Still another aspect of the invention concerns the compounds of formula I. Yet another aspect of the invention concerns the compounds of formula III.

### Detailed description of the invention

### Definitions

In the context of the present invention, the term "C₆₋₂₀ aryl" is intended to mean an optionally substituted fully or partially aromatic carbocyclic ring or ring system with 6 to 20 carbon atoms, such as phenyl, naphthyl, 1,2,3,4-tetrahydronaphthyl, anthracyl, phenanthracyl, pyrenyl, benzopyrenyl, fluorenyl and xanthenyl, among which phenyl is a preferred example.

In the context of the present invention, the term "C₁₋₆ alkyl" is intended to mean a linear or branched saturated hydrocarbon group having from one to six carbon atoms, such as methyl, ethyl, n-propyl, isopropyl, n-butyl, isobutyl, sec-butyl, *tert*-butyl, n-pentyl, isopentyl, neopentyl and n-hexyl.

In the context of the present invention, the term "C₁₋₆-alkoxy" is intended to mean C₁₋₆-alkyl-oxy, such as methoxy, ethoxy, *n*-propoxy, *iso*-propoxy, *n*-butoxy, *iso*-butoxy, *sec*-butoxy, *tert*-butoxy, *n*-pentoxy, *iso*-pentoxy, *neo*-pentoxy and *n-*hexoxy.

In the context of the present invention, the term "C₂₋₆ alkenyl" is intended to cover linear or branched hydrocarbon groups having 2 to 6 carbon atoms and comprising one unsaturated bond. Examples of alkenyl groups are vinyl, allyl, butenyl, pentenyl and hexenyl.

In the context of the present invention, the term "C₃₋₆ cycloalkyl" is intended to mean a cyclic hydrocarbon group having 3 to 6 carbon atoms, such as cyclopropyl, cyclobutyl, cyclopentyl and cyclohexyl.

In the context of the present invention, the term "heteroaryl" is intended to mean a fully or partially aromatic carbocyclic ring or ring system where one or more of the carbon atoms have been replaced with heteroatoms, e.g. nitrogen (=N- or - NH-), sulphur, and/or oxygen atoms. Examples of such heteroaryl groups are oxazolyl, isoxazolyl, thiazolyl, isothiazolyl, pyrrolyl, imidazolyl, pyrazolyl, pyridinyl, pyrimidinyl, pyrazinyl, pyridazinyl, triazinyl, coumaryl, furyl, thienyl, quinolyl, benzothiazolyl, benzotriazolyl, benzodiazolyl, benzooxozolyl, phthalazinyl, phthalanyl, triazolyl, tetrazolyl, isoquinolyl, acridinyl, carbazolyl, dibenzazepinyl, indolyl, benzopyrazolyl, phenoxazonyl, phenyl pyrrolyl and N-phenyl pyrrolyl.

In the present context, the term "optionally substituted" is intended to mean that the group in question may be substituted one or several times, preferably 1-3 times, with group(s) selected from hydroxy (which when bound to an unsaturated carbon atom may be present in the tautomeric keto form), C₁₋₆-alkoxy, C₂₋₆-alkenyloxy, carboxy, oxo (forming a keto or aldehyde functionality), C₁₋₆-alkoxycarbonyl, C₁₋₆-alkylcarbonyl, formyl, aryl, aryloxycarbonyl, aryloxy, arylamino, arylcarbonyl, heteroaryl, heteroarylamino, heteroaryloxycarbonyl, heteroaryloxy, heteroarylcarbonyl, amino, mono- and di(C₁₋₆-alkyl)amino, carbamoyl, mono- and di(C₁₋₆-alkyl)aminocarbonyl, amino-C₁₋₆-alkyl-aminocarbonyl, mono- and di(C₁₋₆-alkyl)amino-C₁₋₆-alkyl-aminocarbonyl, C₁₋₆-alkylcarbonylamino, cyano, guanidino, carbamido, C₁₋₆-alkyl-sulphonyl-amino, aryl-sulphonyl-amino, heteroaryl-sulphonyl-amino, C₁₋₆-alkanoyloxy, C₁₋₆-alkyl-sulphonyl, C₁₋₆-alkyl-sulphinyl, C₁₋₆-alkylsulphonyloxy, nitro, C₁₋₆-alkylthio and halogen.

In the present context, the term "mildly alkaline conditions" refers to conditions created when adding the compound of formula II, which is a base, to the compound of formula I, preferably in the presence of a further base, such as triethylamine, diisopropylethylamine (DIPEA), pyridine, 1,4-diazabicyclo[2.2.2]octane (DABCO), 4-dimethylaminopyridine (DMAP), sodium carbonate, potassium carbonate, sodium hydrogencarbonate, potassium hydrogencarbonate, sodium hydroxide, or potassium hydroxide.

### Processes

In one aspect of the invention, it concerns a process for preparing Indacaterol or a pharmaceutically acceptable salt thereof comprising reacting the compound of formula I with 2-amino-5,6-diethylindan of formula II, preferably in the presence of a base, to the compound of formula III and then converting the compound of formula III to Indacaterol or a pharmaceutically acceptable salt thereof: 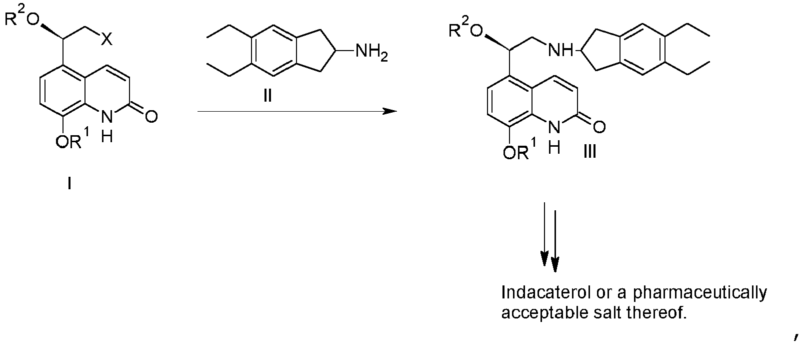 wherein R¹ is benzyl, R² is a protecting group, which is stable under mildly alkaline conditions, and X is a halogen selected from the group consisting of chloro, bromo, and iodo.

In one embodiment, the compound of formula III is converted to Indacaterol by first converting it to a compound of formula IV by first removing the protecting group R² by addition of an acid, preferably an aqueous acid, and finally isolating/purifying the compound (IV) as a salt by adding the acid HA: 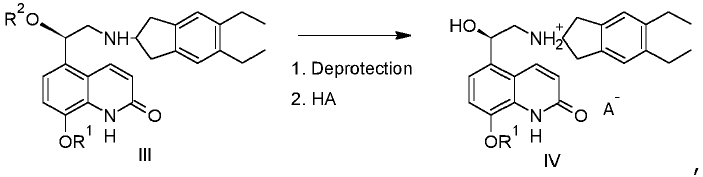 and then converting the compound of formula IV to Indacaterol or a pharmaceutically acceptable salt thereof. Processes for converting the compound of formula IV to Indacaterol or a pharmaceutically acceptable salt thereof are disclosed *inter alia* in WO 2004/076422.

In another aspect of the invention, it concerns a process for the preparation of Indacaterol or a pharmaceutically acceptable salt thereof, comprising precipitating a protected Indacaterol acid salt of formula IV in the presence of water and a water-miscible organic solvent and then converting the precipitated protected Indacaterol acid salt of formula IV to Indacaterol or a pharmaceutically acceptable salt thereof: 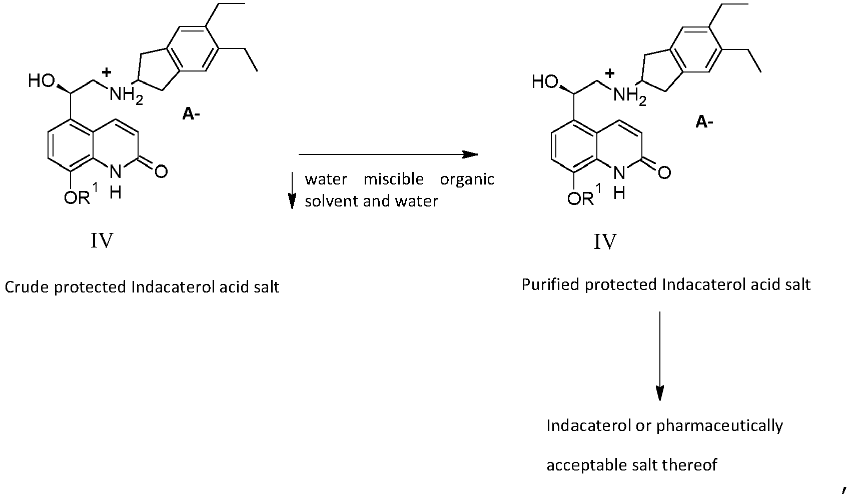 wherein R¹ is benzyl and A⁻ is the counterion of an acid, HA, as defined herein.

In one embodiment, the protected Indacaterol acid salt is formed *in situ* by reacting the protected Indacaterol of formula I with the acid, HA:

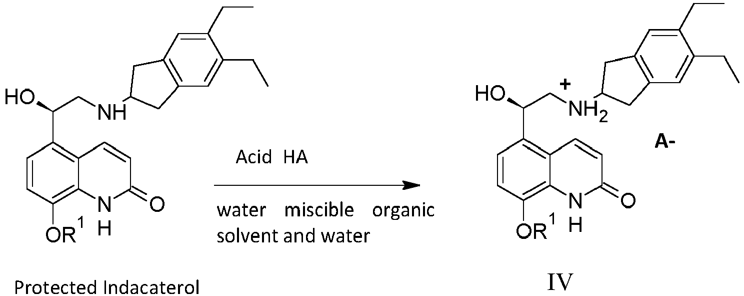

In a further embodiment, the compound of formula IV is converted to Indacaterol or a pharmaceutically acceptable salt thereof by:
a) neutralizing the compound of formula IV, removing benzyl to obtain Indacaterol free base in solution or suspension, optionally isolating Indacaterol free base in solid form, and, optionally, obtaining a pharmaceutically acceptable salt of Indacaterol by addition of a suitable acid, such as maleic acid, to the free base;
b) removing benzyl to obtain a compound of formula V: 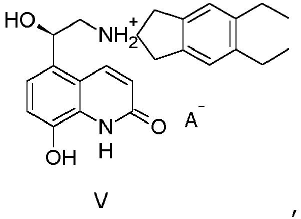 neutralizing the compound of formula V to obtain the free Indacaterol base in solution or suspension, optionally isolating Indacaterol free base in solid form, and, optionally, obtaining a pharmaceutically acceptable salt of Indacaterol by addition of a suitable acid, such as maleic acid, to the free base; or
c) removing benzyl to obtain a compound of formula V, reacting the compound of formula V directly with a suitable acid, such as maleic acid, to obtain a pharmaceutically acceptable salt of Indacaterol.

### The compound of formula III

The compound of formula III may be isolated as the free base or through the formation of an acid addition salt without removing the protecting group R² or used directly without isolating it in the further preparation of Indacaterol or a pharmaceutically acceptable salt therof, such as proceeding via the compound of formula IV.

### R¹ protecting groups

R¹ is a protecting group commonly known in the art for protecting phenol groups. The skilled person will be aware of suitable protecting groups for hydroxy groups in the 8-position of quinolone derivatives such as the compound of formula I. Such suitable protecting groups may be found in WO 00/75114 and WO 2004/076422.

More particularly, in one example, which is not part of the present invention, R¹ is selected from the group consisting of a C₁₋₆ alkyl, C₆₋₂₀ aryl, C₁₋₆-alkoxy, C₂₋₆ alkenyl, C₃₋₆ cycloalkyl, benzocycloalkyl, C₃₋₆ cycloalkyl-C₁₋₆ alkyl, C₆₋₂₀ aryl-C₁₋₆ alkyl, heteroaryl, heteroaryl-C₁₋₆ alkyl, halO-C₁₋₆ alkyl, and an optionally substituted silyl group. In the present invention, R¹ is benzyl.

### R² protecting groups

R² is a protecting group, which is stable under mildly alkaline conditions and which can be cleaved off selectively under conditions where R¹ is not cleaved off. A number of protecting groups fulfil these criteria, including, but not limited to, protecting groups forming an acetal together with the adjacent oxygen atom, protecting groups forming an ether together with the adjacent oxygen, protecting groups forming a silyl ether group with the adjacent oxygen, and protecting groups forming an ester together with the adjacent oxygen. Hence, in one embodiment, R² forms an acetal, an ether, a silyl ether, or an ester together with the adjacent oxygen. In another embodiment, R² forms an acetal, an ether, or a silyl ether together with the adjacent oxygen. In yet another embodiment, R² forms an acetal or an ether together with the adjacent oxygen. In a further embodiment, R² forms an acetal together with the adjacent oxygen.

Examples of suitable acetal protecting groups are 1-(n-butoxy)-ethyl acetal and tetrahydro-pyran-2-yl acetal. Thus, in one embodiment, R² is 1-(n-butoxy)-ethyl or tetrahydro-pyran-2-yl, such as 1-(n-butoxy)-ethyl. Examples of suitable ether protecting groups are benzyl ether, methoxymethyl (MOM) ether, methylthiomethyl (MTM) ether, and benzyloxymethyl ether. Thus, in another embodiment, R² is benzyl, methoxymethyl, methylthiomethyl, or benzyloxymethyl, such as benzyl. Examples of suitable silyl ether protecting groups are trimethylsilyl ether and tert-butyldimethylsilyl ether. Thus, in still another embodiment, R² is trimethylsilyl or tert-butyldimethylsilyl. Examples of suitable ester protecting groups are pivaloyl ester and acetate ester. Thus, in yet another embodiment, R² is pivaloyl or acetate.

In a further embodiment, R² is selected from the group consisting of 1-(n-butoxy)-ethyl, methoxymethyl, benzyl, and tetrahydro-pyran-2-yl, such as from the group consisting of 1-(n-butoxy)-ethyl, methoxymethyl, and tetrahydropyran-2-yl. In yet a further embodiment, R² is 1-(n-butoxy)-ethyl and R¹ is benzyl.

### Methods for removing the protecting group R²

The protecting group R² may be removed from the compound of formula III by methods known in the art for the various R² protecting groups defined herein. In the case of R² forming an acetal together with the adjacent oxygen atom, R² may be removed by reacting with an intermediate to strong acid, preferably in the presence of water. Examples of suitable acids are hydrochloric acid, hydrobromic acid, sulfuric acid, nitric acid, phosphoric acid, camphorsulfonic acid, methanesulfonic acid, trifluoromethanesulfonic acid, and combinations thereof.

In the case of R² forming an ether, silyl ether, or ester together with the adjacent oxygen atom, the acids mentioned for the acetal protecting groups are also suitable for removing R². Other suitable agents for removing R² in the case of R² forming an ether, silyl ether, or ester together with the adjacent oxygen atom are aqueous bases, lewis acids, hydrogen over palladium or platinum catalyst (in the case of benzyl ether), resins such as Dowex, thiols such as thiophenol, and combinations thereof.

### Bases useful in the reaction of compounds I and II

Any organic or inorganic base may be employed in the reaction between compounds I and II in the formation of the compound of formula III, with the exception of primary and secondary amines. Examples of useful organic bases in this reaction are triethylamine, diisopropylethylamine (DIPEA), pyridine, 1,4-diazabicyclo[2.2.2]octane (DABCO), and 4-dimethylaminopyridine (DMAP). Examples of useful inorganic bases in this reaction are sodium carbonate, potassium carbonate, sodium hydrogencarbonate, potassium hydrogencarbonate, sodium hydroxide, and potassium hydroxide. When carrying out the reaction between the compounds of formula I and II in the presence of a base, the 2-amino-5,6-diethylindan of formula II may be added to the reaction mixture in the form of an acid addition salt thereof, such as the hydrochloride salt thereof.

### The acid HA

Reacting the product obtained by removing the protecting group R² from the compound of formula III with the acid HA serves to purify the compound by obtaining the salt of formula IV. Examples of suitable HA acids are benzoic acid, maleic acid, fumaric acid, succinic acid, tartaric acid, hydrochloric acid, hydrobromic acid, dibenzoyl-tartaric acid, mandelic acid, and camphorsulfonic acid.

In one embodiment, the acid HA is selected from the group consisting of tartaric acid, dibenzoyl-tartaric acid, mandelic acid, succinic acid, and benzoic acid. In another embodiment, the acid HA is selected from the group consisting of tartaric acid, succinic acid, and benzoic acid.

In another embodiment, the acid HA is selected from the group consisting of L-tartaric acid and dibenzoyl-L-tartaric acid.

### The mixture of water and water-miscible organic solvent

It has been found that a mixture of water and a water-miscible organic solvent rather than the water-miscible organic solvent alone provides a high enantiomeric purity of the final product. In one embodiment, the water-miscible organic solvent is selected from the group consisting of methanol, ethanol, isopropyl alcohol, acetone, acetonitrile, and mixtures thereof. In a further embodiment, the water-miscible organic solvent is selected from the group consisting of acetone, ethanol, and mixtures thereof.

### The halogen X

Halogens generally constitute good leaving groups in an S_{N}2-type reaction, such as the reaction between the compounds of formula I and II. In one embodiment, X is selected from the group consisting of chloro, bromo, and iodo. In another embodiment, X is bromo or iodo. In yet another embodiment, X is bromo.

In a further embodiment, X is bromo or chloro and the reaction between compounds I and II takes place in the presence of an iodine salt, such sodium iodide or potassium iodide, which generates the iodo group *in situ.*

### The starting compound of formula I

The compound of formula I may be obtained from the corresponding hydroxy-unprotected compound of formula VI: 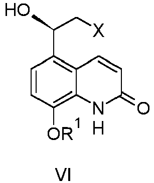 by reacting with the reagents known in the art to form the acetal, ether, silyl ether, or ester protecting groups defined herein when reacted with an alcohol. In the case of e.g. acetal protecting groups, in the case where R² is 1-(n-butoxy)-ethyl or tetrahydro-pyran-2-yl, the compound of formula VI may be reacted with butyl-vinyl ether or dihydro-pyran-2-yl, respectively.

The compound of formula VI may be prepared by reducing the corresponding haloacetyl compound using a chiral catalyst. Suitable chiral catalysts for this method are disclosed in WO 2004/076422 and WO 2005/123684.

### Pharmaceutically acceptable salts

Pharmaceutically acceptable acid addition salts of Indacaterol are easily identified by the skilled person. A useful list of pharmaceutically acceptable acid addition salts may be found in Berge et al: "Pharmaceutical Salts", Journal of Pharmaceutical Sciences, vol. 66, no. 1, 1 January 1977, pages 1-19. A particularly interesting pharmaceutically acceptable acid addition salt is the maleate salt.

### Proceeding via Indacaterol base

As discussed above, Indacaterol free base is known in the art to be unstable in organic solvents. Hence, preparing pharmaceutically acceptable salts of Indacaterol by proceeding via the free Indacaterol base is not considered viable on an industrial scale. It has, however, been found that by isolating the free base in solid form, pharmaceutically acceptable salts of Indacaterol may indeed be prepared on an industrial scale by proceeding via the free Indacaterol base. Furthermore, this avoids the impurities associated with the methods known in the art for converting one salt of 8-protected Indacaterol directly to a pharmaceutically acceptable salt of Indacaterol. Example 2 of WO 2004/076422 was reproduced, hydrogenating the benzoate salt of formula IV using acetic acid as the solvent, and then exchanging the anion of the salt to maleate by addition of maleic acid. The obtained solid was filtered, washed, and dried in vacuum to give the Indacaterol maleate with impurities of Indacaterol acetate as measured by NMR (Comparative example 9).

Thus, in another aspect of the invention, it concerns a process for the preparation of a pharmaceutically acceptable salt of Indacaterol by obtaining Indacaterol, isolating it in solid form, and reacting it with a suitable acid, such as maleic acid. Indacaterol free base may be obtained as disclosed herein or as known in the art.

### Useful reaction conditions

### Formation of the compound of formula III

The reaction may take place in a number of different organic solvents. Useful examples are acetonitrile, butanone, and dimethylformamide (DMF), in particular acetonitrile and butanone. It has been found advantageous to use small volumes of solvent in the reaction between the compounds of formula I and II. The reaction is advantageously carried out at a temperature in the range of 70 to 110°C, such as at 85°C, with a duration of between 2 and 10 hours, such as 4 to 5 hours. Furthermore, when adding the 2-amino-5,6-diethylindan of formula II as an acid addition salt thereof, a carbonate salt, such as potassium carbonate, is advantageously added to the reaction mixture.

### Removing the protecting group R²

When using an aqueous acid for removing the protecting group R², e.g. 1-(n-butoxy)-ethyl, from the compound of formula III said acid, such as hydrochloric acid, is advantageously added in excess, such as 2 to 6 equivalents, at a temperature between room temperature and reflux until complete removal of the protecting group, e.g. 1 to 3 hours for removing the 1-(n-butoxy)-ethyl protecting group.

### Formation of the compound of formula IV

Once the protecting group R² has been removed, more water may advantageously be added together with a suitable solvent, such as dichloromethane. The deprotected compound may be neutralized at a pH of 9 to 11 and the resulting phases then separated. After separation, the solvent may be changed to a solvent suitable for precipitation of the compound of formula IV. Useful solvents are ethyl acetate, isopropanol, ethanol, acetone, tetrahydrofuran, and acetonitrile, ethyl acetate, isopropanol, and ethanol currently being more preferred. After changing the solvent, the acid HA may be added to form the compound of formula IV by precipitation. Ethyl acetate is a particularly useful solvent for precipitating the benzoate, succinate, and tartrate salts. The salt of formula IV may be obtained with a yield of 65 to 80% and a purity of greater than 93%% in the case of tartrate precipitated in ethyl acetate, and a yield of 60 to 75% and a purity of greater than 99% in the case of succinate and tartrate precipitated in isopropanol or ethanol. The absence of dimer and regioisomer impurities as known in the art facilitates a more quantitative precipitation using ethyl acetate since there is no competition for the base molecules.

### Formation of Indacaterol base

The compound of formula IV may be neutralized before deprotection of R¹. The neutralization may suitably be achieved by addition of dichloromethane, water and soda. When R¹ is removed by hydrogenation, it may suitably be achieved using an overpressure of hydrogen at ambient temperature. Furthermore, a mixture of methanol and dichloromethane as the solvent is suitably employed in the process. Upon completion of the hydrogenation, the catalyst is removed and dichloromethane is distilled off to leave methanol as the only solvent, which causes Indacaterol to precipitate upon cooling. Alternatively, the methanol/dichloromethane mixture is exchanged with isopropanol solvent, which is cooled to achieve precipitation of Indacaterol base with a purity of > 99%.

Precipitated Indacaterol base is a white solid, which may be stored at ambient temperature for extended periods of time. Upon dissolution it may be used to prepare a pharmaceutically acceptable salt, such as the maleate salt. A suitable solvent for the addition of maleic acid is isopropanol. Alternatively, Indacaterol base obtained from the reaction and dissolved in a mixture of methanol and dichloromethane can be used directly, the solvent exchanged for isopropanol, and then precipitated as the maleate salt by adding maleic acid.

### Intermediate compounds

The process of the invention involves novel intermediates, which have not previously been used in the preparation of Indacaterol. Hence, a further aspect of the invention concerns the compounds of formula I, with the proviso that when R¹ is benzyl and X is Br, then R² is not tert-butyl(dimethyl)silyl or tetrahydro-2H-pyran-2-yl.

Yet another aspect of the invention concerns the compounds of formula III, or salts thereof.

### Examples

### Example 1 - protecting the α-halohydroxy compound of formula VI

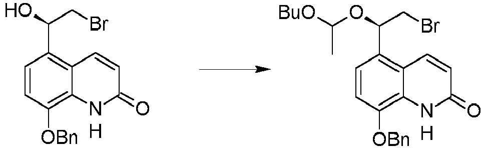

A flask is charged with 5 ml of tetrahydrofuran (THF) and 5 ml of toluene. p-toluene sulfonic acid (0,15 mmol) and molecular sieves are added with stirring for 30 minutes. 6 mmol of butyl-vinylether and 3 mmol of 8-(phenylmethoxy)-5-((*R*)-2-bromo-1-hydroxy-ethyl)-(1*H*)-quinolin-2-one are added. The mixture is agitated at 20/25° C until completion of the reaction, followed by filtration and distillation of the filtrate to remove the solvent. The product is obtained in quantitative yield as an oil consisting of 50% of each of the diastereomers.

¹H-NMR (DMSO-*d6*, δ), mixture 50/50 of diastereomers: 0.61 and 0.82 (3H, t, J=7.2 Hz, CH₃-Pr-O), 1.12 and 1.22 (3H, d, J=5.6 Hz, acetalic CH₃), 0.90-1.40 (4H, m, CH₂ + CH₂), 3.20-3.80 (4H, m, CH₂-OAr + CH₂-Br), 4.51 and 4.82 (1H, q, J=5.6 Hz, acetalic CH), 5.18 and 5.24 (1H, dd, J=4.0, 8.0 Hz, CH-O-acetal), 6.56 and 6.58 (1H, d, J=10.0 Hz, H4), 7.00-7.57 (7H, m), 8.17 and 8.23 (1H, d, J=10.0 Hz, H3), 10.71 (1H, s, NH)

¹³C-NMR (DMSO-*d6*, δ), mixture 50/50 of diastereoisomers: 13.5 and 13.7 CH₃), 18.5 and 18.8 (CH₂), 19.9 and 20.0 (acetalic CH₃), 30.9 and 31.4 (CH₂), 36.8 and 37.3 (CH₂), 63.7 and 64.2 (CH₂-Br), 69.8 and 69.9 (CH₂-OAr), 73.8 and 75.1 (CHO), 97.5 and 100.4 (acetalic CH), 111.8 (CH), 116.9 and 117.2 (C), 121.2 and 122.4 (CH), 122.3 and 122.6 (CH), 127.7 and 127.8 (C), 127.8 and 127.9 (CH), 128.2 and 128.3 (CH), 128.8 and 129.1 (C), 129.4 and 129.6 (C), 136.1 and 136.5 (CH), 136.5 and 136.6 (C), 144.0 and 144.2 (C), 160.7 and 160.8 (C=O).

### Example 2 - protecting the α-halohydroxy compound of formula VI

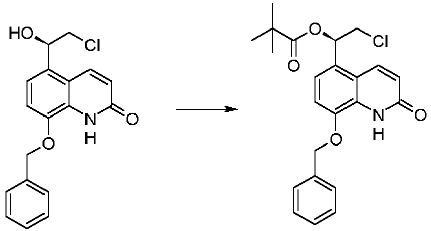

Pivaloyl chloride (0.72 g) is added to a stirred mixture of 8-(phenylmethoxy)-5-((*R*)-2-chloro-1-hydroxy-ethyl)-(1*H*)-quinolin-2-one (0.74 g), dichloromethane (15 ml) and 4-dimethylaminopyridine (0.89 g) at 20/25° C, and the reaction is stirred until all the starting material disappeared. Water (22 ml) is added and the phases are separated.

The organic phase is washed with 1 M HCl (22 ml) and then with water (22 ml). The solvent is removed and the residue is crystallized from acetone to obtain 0.82 g of the product.

¹H-NMR (DMSO-*d6*, δ): 1.13 (9H, s, CH₃), 3.92 (1H, dd, J= 4.0, 12.0 Hz, CH₂-Br), 4.00 (1H, dd, J= 8.4, 12.0 Hz, CH₂-Cl), 5.28 (2H, s, Ph-CH₂-O), 6.25 (1H, dd, J= 4.0, 8.4 Hz, CH-OPiv), 6.59 (1H, d, J= 10.0 Hz, H4), 7.15 (1H, d, J= 8.4 Hz, H6), 7.20 (1H, d, J= 8.4 Hz, H7), 7.27-7.30 (1H, m, Ph), 7.33-7.37 (2H, m, Ph), 7.54-7.56 (2H, m, Ph), 8.18 (1H, d, J= 10.0 Hz, H3), 10.77 (1H, s, NH).

¹³C-NMR (DMSO-*d6*, δ): 26.7 (3 x CH₃), 38.3 (C), 46.4 (CH₂-Cl), 69.8 (CH₂-Ph), 71.3 (CH-OPiv), 111.9 (CH), 116.8 (C), 120.5 (CH), 122.9(CH), 126.0 (C), 127.8 (2 x CH), 127.9 (CH), 128.3 (2 x CH), 129.5 (C), 136.0 (C), 136.5 (CH), 144.5 (C), 160.7 (CON), 176.2 (COO).

### Example 3 - preparation of the compound of formula IV

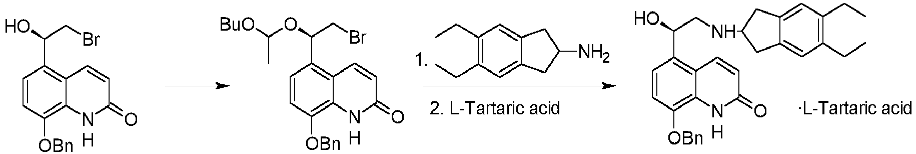

A flask is charged with 2.5 ml of THF and 2.5 ml of toluene. p-toluene sulfonic acid (5 mg) and molecular sieves (0.2 g) are added with stirring for 30 minutes. 1.5 ml of butyl-vinylether and 2 g of 8-(phenylmethoxy)-5-((*R*)-2-bromo-1-hydroxy-ethyl)-(1*H*)-quinolin-2-one are added. The mixture is agitated at 20/25° C until completion of the reaction. 0.015 ml of diisopropylethyl amine is added, the mixture is filtered, and the solvent is distilled off.

The residue is dissolved in 6 ml of dimethylformamide (DMF), 1.9 ml of diisoproypylethyl amine, 1.2 g sodium iodide, and 1.5 g of 2-amino-5,6-diethylindane are added and the mixture is heated to 100° C. After completion of the reaction the mixture is cooled to 20/25° C, 0.4 ml of concentrated hydrochloric acid and 0.4 ml of water are added, and the mixture is stirred for 30 minutes.

HPLC analysis shows the expected product with a purity of 75% and being free from the dimer and regioisomer impurities.

20 ml of water, 20 ml of methylene chloride, and 3 ml of 6N NaOH are added with stirring. The organic phase is separated and washed with 20 ml of water. The organic phase is distilled and the solvent is changed to ethyl acetate with a final volume of 100 ml. The mixture is heated to 70° C, 0.8 g of L-tartaric acid is added, and stirring continues for 30 minutes at 70° C. The mixture is cooled slowly to 20/25° C, filtered, and washed with 8 ml of ethyl acetate to obtain 8-(phenylmethoxy)-5-[(*R*)-2-(5,6-diethyl-indan-2-ylamino)-1-hydroxy-ethyl]-(1*H*)-quinolin-2-one tartrate in 68% yield. The purity of the product is >95% by HPLC analysis.

### Example 4 - preparation of the compound of formula IV

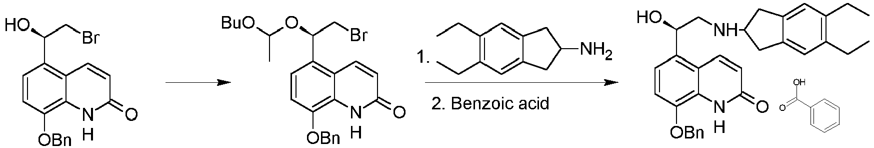

A flask is charged with 19 ml of THF and 19 ml of toluene. p-toluene sulfonic acid (75 mg) and molecular sieves (1.5 g) are added and the mixture is stirred for 30 minutes. 11.2 ml of butyl-vinylether and 15 g of 8-(phenylmethoxy)-5-((*R*)-2-bromo-1-hydroxy-ethyl)-(1*H*)-quinolin-2-one are added. The mixture is agitated at 20/25° C until completion of the reaction. 0.1 ml of diisopropylethyl amine are added, the mixture is filtered, and the solvent is distilled off.

The residue is dissolved in 40 ml of butanone, 14.5 ml of diisoproypylethyl amine, 9 g sodium iodide, and 11.3 g of 2-amino-5,6-diethylindane are added and the mixture is heated to 90-100° C. After completion of the reaction the mixture is cooled to 20/25° C, 3 ml of concentrated hydrochloric acid and 3 ml of water are added, and the mixture is stirred for 30 minutes.

HPLC analysis shows the expected product with a purity of 84% and being free from the dimer and regioisomer impurities.

150 ml of water, 150 ml of methylene chloride, and 22.5 ml of 6N NaOH are added with stirring. The organic phase is separated and washed with 10 ml of water. The organic phase is distilled and the solvent is changed to isopropyl alcohol with a final volume of 300 ml. The mixture is heated to 70° C, 4.9 g of benzoic acid is added, and stirring continues for 30 minutes at 70° C. The mixture is cooled slowly to 20/25° C, filtered, and washed with 30 ml of isopropanol to obtain 8-(phenylmethoxy)-5-[(*R*)-2-(5,6-diethyl-indan-2-ylamino)-1-hydroxyethyl]-(1*H*)-quinolin-2-one benzoate in 59 % yield. The purity of the product is > 99 % by HPLC analysis.

### Example 5 - preparation of the compound of formula IV

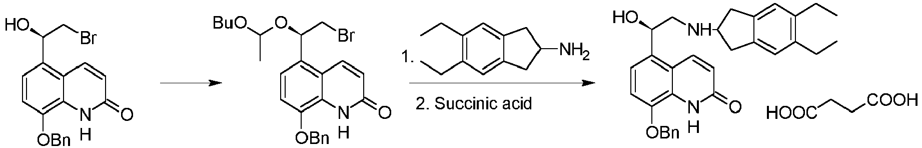

A flask is charged with 7.5 ml of THF and 7.5 ml of toluene. p-toluene sulfonic acid (30 mg) and molecular sieves (0.6 g) are added and the mixture is stirred for 30 minutes. 4.5 ml of butyl-vinylether and 6 g of 8-(phenylmethoxy)-5-((*R*)-2-bromo-1-hydroxy-ethyl)-(1*H*)-quinolin-2-one are added. The mixture is agitated at 20/25° C until completion of the reaction. 0.040 ml of diisopropylethyl amine are added, the mixture is filtered, and the solvent is distilled off.

The residue is dissolved in 18 ml of acetonitrile (ACN), 5,8 ml of diisoproypylethyl amine, 3.6 g sodium iodide, and 4.5 g of 2-amino-5,6-diethylindane are added and the mixture is heated to 80-90° C. After completion of the reaction the mixture is cooled to 20/25° C, 1.2 ml of concentrated hydrochloric acid and 1.2 ml of water are added, and the mixture is stirred for 30 minutes. HPLC analysis shows the expected product with a purity of 89% and being free from the dimer and regioisomer impurities.

60 ml of water, 60 ml of methylene chloride, and 9 ml of 6N NaOH are added with stirring. The organic phase is separated and washed with 60 ml of water. The organic phase is distilled and the solvent is changed to isopropyl alcohol with a final volume of 120 ml. The mixture is heated to 70° C, 1.9 g of succinic acid is added, and stirring continues for 30 minutes at 70° C. The mixture is cooled slowly to 20/25° C, filtered, and washed with 12 ml of isopropanol to obtain 8-(phenylmethoxy)-5-[(*R*)-2-(5,6-diethyl-indan-2-ylamino)-1-hydroxy-ethyl]-(1*H*)-quinolin-2-one succinate in 56 % yield. The purity of the product is > 99 % by HPLC analysis.

### Example 6: purification with EtOH/water

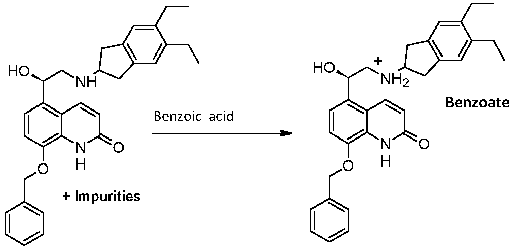

To 2.0 g of 8-(phenylmethoxy)-5-[(R)-2-(5,6-diethyl-indan-2-ylamino)-1-hydroxy-ethyl]-(1H)-quinolin-2-one, a mixture of 35 ml/g of EtOH and 5 ml/g of water are added and heated to reflux. Once this temperature is reached, benzoic acid is added (1.2 eq.) as a solution in 5 ml/g of the mixture of EtOH/water. The temperature is maintained for 30 minutes. The mixture is then cooled slowly overnight to 20-25°C. The resulting suspension is filtered and a white solid is obtained and dried in vacuum. The white solid is analyzed by HPLC to determine the chromatographic purity and by chiral HPLC to determine the enantiomeric purity, obtaining a white solid product with a proportion of enantiomeric impurity below 0.05%. No other impurities are detected.

### Example 7: purification with Acetone/water

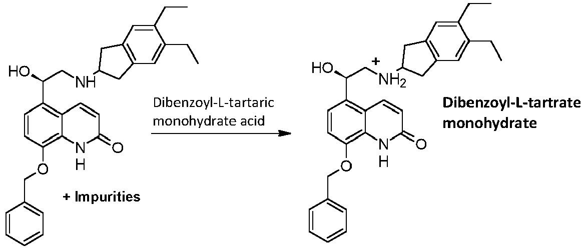

To 2.0 g of 8-(phenylmethoxy)-5-[(R)-2-(5,6-diethyl-indan-2-ylamino)-1-hydroxy-ethyl]-(1H)-quinolin-2-one, a mixture of 35 ml/g of Acetone and 1 ml/g of water are added and heated to reflux. Once this temperature is reached, Dibenzoyl-L-tartaric monohydrate acid is added (1.2 eq.) as a solution in 5 ml/g of the mixture of Acetone /water. The temperature is maintained for 30 minutes. The mixture is then cooled slowly overnight to 20-25°C. The resulting suspension is filtered and a white solid is obtained and dried in vacuum. The white solid is analyzed by HPLC to determine the chromatographic purity and by chiral HPLC to determine the enantiomeric purity, obtaining a white solid product with a proportion of enantiomeric impurity below 0.05%. No other impurities are detected.

### Example 8: purification with EtOH/water

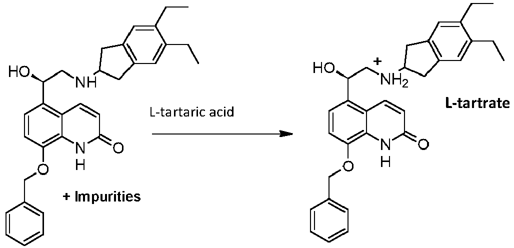

To 2.0 g of of 8-(phenylmethoxy)-5-[(R)-2-(5,6-diethyl-indan-2-ylamino)-1-hydroxy-ethyl]-(1H)-quinolin-2-one, a mixture of 35 ml/g of EtOH and 5 ml/g of water are added and heated to reflux. Once this temperature is reached, L Tartaric acid is added (1.2 eq.) as a solution in 5 ml/g of the mixture of EtOH/water. The temperature is maintained for 30 minutes. The mixture is then cooled slowly overnight to 20-25°C. The resulting suspension is filtered and a white solid is obtained and dried in vacuum. The white solid is analyzed by HPLC to determine the chromatographic purity and by chiral HPLC to determine the enantiomeric purity, obtaining a white solid product with a proportion of enantiomeric impurity below 0.06%. No other impurities are detected.

### Example 9: synthesis of protected benzyl Indacaterol

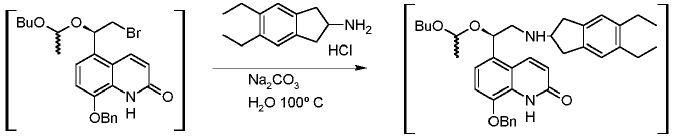

A solution of sodium carbonate (0.57 kg/kg, 2 equivalents) in water (13 l/kg) is prepared in another reactor. This carbonate solution is added to the product solution from example 1, diethyl indanolamine·HCl (0.72 kg/kg, 1.2 equivalents) is added and the mixture is heated and distilled at atmospheric pressure until a volume of 13 l/kg. Water (3 l/kg) is added and the mixture is distilled at atmospheric pressure until a volume of 13 l/kg. The system is placed in reflux position and reflux is maintained for 20 hours.

When the reaction is complete, the mixture is cooled to 20-25°C and methylene chloride (15 l/kg) is added. The mixture is agitated, decanted, and the aqueous phase is extracted with methylene chloride (5 l/kg). The organic phases are washed with water (5 l/kg).

### Example 10 - preparation of Indacaterol maleate

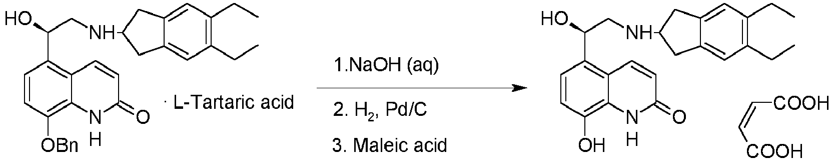

28 g of 8-(phenylmethoxy)-5-[(*R*)-2-(5,6-diethyl-indan-2-ylamino)-1-hydroxyethyl]-(1*H*)-quinolin-2-one tartrate is dissolved in a mixture of 560 ml of dichloromethane, 560 ml of water, and 30 ml of an aqueous solution of 6N sodium hydroxide under stirring. The phases are separated and the organic phase is washed with 280 ml of water.

The organic phase is distilled to a final volume of 140 ml and 420 ml of methanol and 4.2 g of Pd/C (5% - 50% water) are added. The system is purged with nitrogen and subsequently with hydrogen at an overpressure of 0.3 bar and stirring until completion of the reaction.

The catalyst is filtered off and the solvent is changed to isopropanol adjusting the final volume to 950 ml. The solution is heated to 70/80° C and a solution of 5.4 g maleic acid in 140 ml of isopropanol is added, maintaining the temperature between 70 and 80° C. The mixture is stirred at 70/80° C for 30 minutes and then slowly cooled to 20/25° C. The resulting suspension is filtered, the solid residue is washed with 90 ml of isopropanol and dried to obtain 18g of Indacaterol maleate (Yield: 79%). The product shows 99.6% purity by HPLC analysis.

### Example 11 - Isolation of Indacaterol free base in solid form

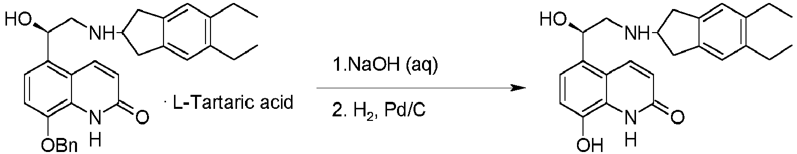

1g of 8-(phenylmethoxy)-5-[(*R*)-2-(5,6-diethyl-indan-2-ylamino)-1-hydroxyethyl]-(1*H*)-quinolin-2-one tartrate is dissolved in a mixture of 20 ml of dichloromethane,20 ml of water, and1 ml of an aqueous solution of 6N sodium hydroxide under stirring. The phases are separated and the organic phase is washed with 10 ml of water.

The organic phase is distilled to a final volume of 5 ml and 15 ml of methanol and 0.15 g of Pd/C (5% - 50% water) are added. The system is purged with nitrogen and subsequently with hydrogen at an overpressure of 0.3 bar and stirring until completion of the reaction.

The catalyst is filtered off and the solvent is changed to isopropanol adjusting the final volume to 8 ml. The resulting suspension is cooled to 0-5°C, filtered and the solid residue is washed with isopropanol and dried to obtain 0.47 g of Indacaterol free base (77%) showing 99.6% purity by HPLC analysis.

A sample of Indacaterol free base stored at 20-25°C is analysed one month later without showing any loss of purity.

### Example 12 - obtaining the maleate salt from Indacaterol free base

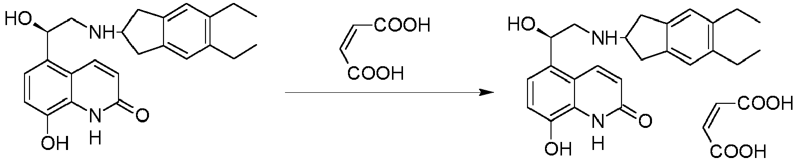

0.47 g of solid Indacaterol are suspended in 20 ml of isopropanol, heated to 70/80° C, and a solution of 0.15 g of maleic acid in 5 ml of isopropanol are added, maintaining the temperature between 70 and 80° C. The mixture is cooled to 0/5°C and filtration of the resulting solid affords 0.52 g of Indacaterol maleate with a purity of 99.7%.

### Comparative example 13 - direct conversion to Indacaterol maleate

8-(phenylmethoxy)-5-[(*R*)-2-(5,6-diethyl-indan-2-ylamino)-1-hydroxy-ethyl]-(1*H*)-quinolin-2-one benzoate (4 g) is dissolved in acetic acid (40 ml). Pd/C (5 %, 50% wet, 0.6 g) is added and the product is hydrogenated under a hydrogen atmosphere. When the reaction is complete the catalyst is filtered off and the filtrate is vacuum distilled until a volume of 8 ml is reached.

Ethanol (40 ml) is added and the mixture is heated to 50° C. A solution of 1.2 g of maleic acid in 2.4 ml of ethanol is added and the mixture is seeded with indacaterol maleate and then slowly cooled to 0/5° C. The solid is filtered and washed with 5 ml of ethanol and 3 ml of isopropanol to obtain 6.0 g of indacaterol maleate.

1H-NMR analysis of the solid shows the presence of acetic acid in 2-4 % by integration of the peak at δ 1.88 (400 MHz, DMSO-*d6*) corresponding to acetic acid.

## Claims

1. A process for the preparation of Indacaterol or a pharmaceutically acceptable salt thereof, comprising reacting the compound of formula I with 2-amino-5,6-diethylindan of formula II, preferably in the presence of a base, to the compound of formula III and then converting the compound of formula III to Indacaterol or a pharmaceutically acceptable salt thereof: 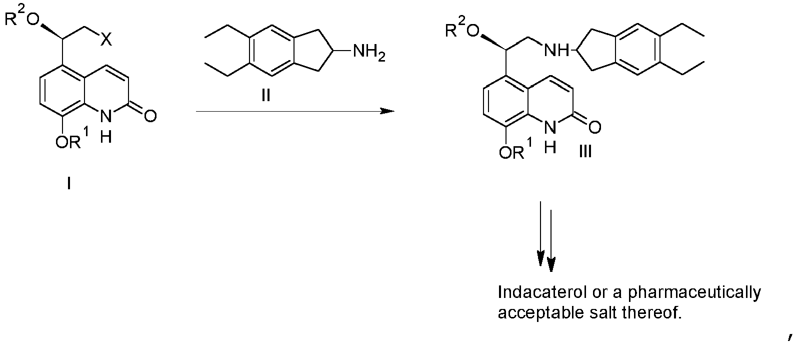 wherein R¹ is benzyl, R² is a protecting group, which is stable under mildly alkaline conditions, and X is a halogen selected from the group consisting of chloro, bromo, and iodo.

2. The process according to claim 1, wherein the compound of formula III is converted to Indacaterol by first converting it to a compound of formula IV by first removing the protecting group R² by addition of an acid, preferably an aqueous acid, and then adding the acid HA: 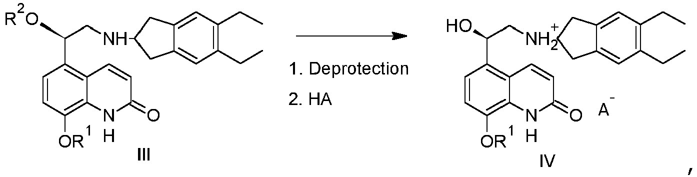 and then converting the compound of formula IV to Indacaterol or a pharmaceutically acceptable salt thereof.

3. The process according to claim 2, further comprising precipitating a protected Indacaterol acid salt of formula IV in the presence of water and a water-miscible organic solvent and then converting the precipitated protected Indacaterol acid salt of formula IV to Indacaterol or a pharmaceutically acceptable salt thereof:

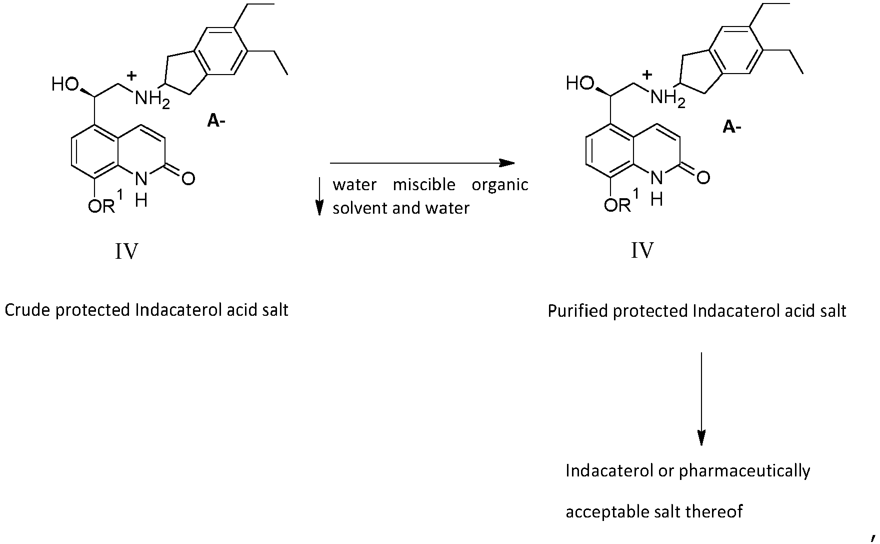

4. The process according to claim 1 to 3, wherein the compound of formula IV is converted to Indacaterol or a pharmaceutically acceptable salt thereof by:
a) neutralizing the compound of formula IV, removing benzyl to obtain Indacaterol free base in solution or suspension, optionally isolating Indacaterol free base in solid form and, optionally, obtaining a pharmaceutically acceptable salt of Indacaterol by addition of a suitable acid, such as maleic acid, to the free base;
b) removing benzyl to obtain a compound of formula V: 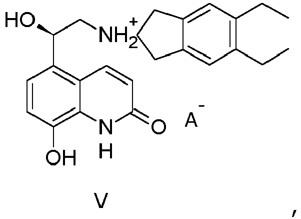 neutralizing the compound of formula V to obtain the free Indacaterol base, in solution or suspension, optionally isolating Indacaterol free base in solid form and, optionally, obtaining a pharmaceutically acceptable salt of Indacaterol by addition of a suitable acid, such as maleic acid, to the free base; or
c) removing benzyl to obtain a compound of formula V, reacting the compound of formula V directly with a suitable acid, such as maleic acid, to obtain a pharmaceutically acceptable salt of Indacaterol.

5. The process according to any one of the preceding claims, wherein R² forms an acetal, an ether, a silyl ether, or an ester together with the adjacent oxygen.

6. The process according to any one of the preceding claims, wherein R² is selected from the group consisting of 1-(n-butoxy)-ethyl and tetrahydro-pyran-2-yl.

7. The process according to any one of the preceding claims, wherein the acid HA is selected from the group consisting of L-tartaric acid, dibenzoyl-L-tartaric acid, mandelic acid, succinic acid and benzoic acid.

8. The process according to any one of the preceding claims, wherein X is chloro or bromo, and wherein an iodide salt, such as sodium iodide, is added in the step reacting the compounds of formula I and II.

9. The process according to any one of the preceding claims, wherein the final product of the process is the maleate salt of Indacaterol.

10. The process according to any one of the preceding claims for the preparation of a pharmaceutically acceptable salt of Indacaterol, said process comprising obtaining Indacaterol free base, isolating it in solid form, and reacting it with a suitable acid, such as maleic acid.

11. A compound of formula I: 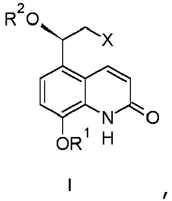 wherein R¹, R², and X are as defined in any one of claims 1 to 10, with the proviso that when R¹ is benzyl and X is Br, then R² is not tert-butyl(dimethyl)silyl or tetrahydro-2H-pyran-2-yl.

12. A compound of formula III: 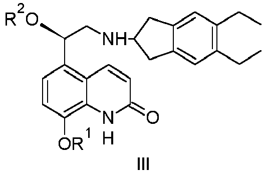 or a salt thereof, wherein R¹ and R² are as defined in any one of claims 1 to 10.

## Patentansprüche

1. Verfahren zur Herstellung von Indacaterol oder einem pharmazeutisch annehmbaren Salzes davon, umfassend Umsetzen der Verbindung der Formel I mit 2-Amino-5,6-diethylindan der Formel II, vorzugsweise in Gegenwart einer Base, zur Verbindung der Formel III und dann Umwandeln der Verbindung der Formel III zu Indacaterol oder einem pharmazeutisch annehmbaren Salz davon: 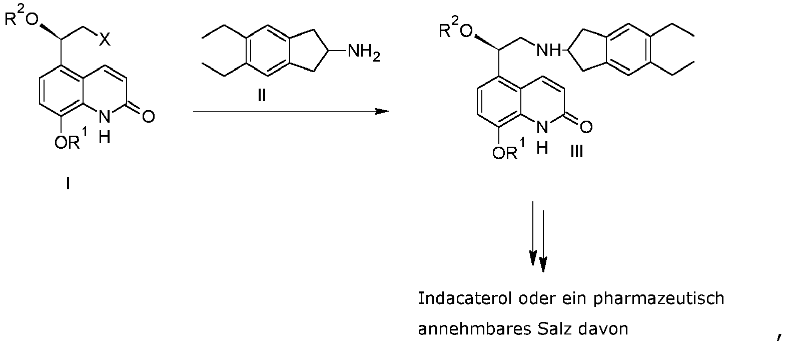 wobei R¹ Benzyl ist, R² eine Schutzgruppe ist, die unter leicht alkalischen Bedingungen stabil ist, und X ein Halogen ist, das aus der Gruppe ausgewählt ist, die aus Chlor, Brom und Iod besteht.

2. Verfahren nach Anspruch 1, wobei die Verbindung der Formel III zu Indacaterol umgewandelt wird, indem sie zuerst zu einer Verbindung der Formel IV umgewandelt wird, indem zuerst die Schutzgruppe R² durch Zugabe einer Säure, vorzugsweise einer wässrigen Säure, entfernt wird und dann die Säure HA zugegeben wird: 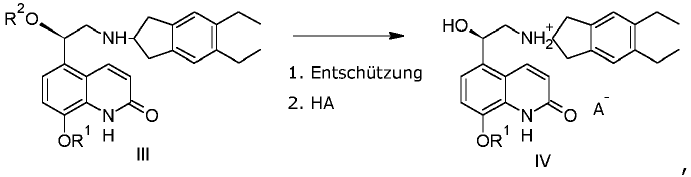 und dann die Verbindung der Formel IV zu Indacaterol oder einem pharmazeutisch annehmbaren Salz davon umgewandelt wird.

3. Verfahren nach Anspruch 2, ferner umfassend Ausfällen eines geschützten Indacaterolsäuresalzes der Formel IV in Gegenwart von Wasser und einem mit Wasser mischbaren organischen Lösungsmittel und dann Umwandeln des ausgefällten geschützten Indacaterolsäuresalzes der Formel IV zu Indacaterol oder einem pharmazeutisch annehmbaren Salz davon:

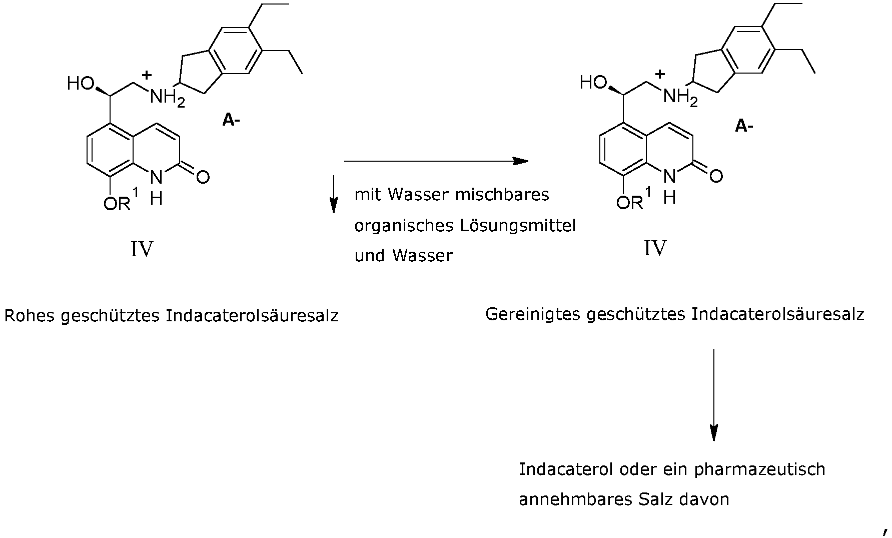

4. Verfahren nach Anspruch 1 bis 3, wobei die Verbindung der Formel IV durch Folgendes zu Indacaterol oder einem pharmazeutisch annehmbaren Salz davon umgewandelt wird:
a) Neutralisieren der Verbindung der Formel IV, Entfernen von Benzyl, um Indacaterol in Form einer freien Base in Lösung oder Suspension zu erhalten, gegebenenfalls Isolieren von Indacaterol in Form der freien Base in fester Form und gegebenenfalls Erhalten eines pharmazeutisch annehmbaren Salzes von Indacaterol durch Zugabe einer geeigneten Säure, wie etwa Maleinsäure, zu der freien Base;
b) Entfernen von Benzyl, um eine Verbindung der Formel V zu erhalten: 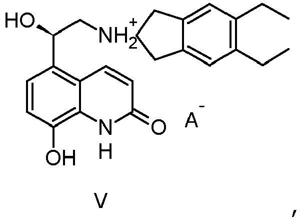 Neutralisieren der Verbindung der Formel V, um die freie Indacaterol-Base in Lösung oder Suspension zu erhalten, gegebenenfalls Isolieren von Indacaterol in Form der freien Base in fester Form und gegebenenfalls Erhalten eines pharmazeutisch annehmbaren Salzes von Indacaterol durch Zugabe einer geeigneten Säure, wie etwa Maleinsäure, zu der freien Base; oder
c) Entfernen von Benzyl, um eine Verbindung der Formel V zu erhalten, Umsetzen der Verbindung der Formel V direkt mit einer geeigneten Säure, wie etwa Maleinsäure, um ein pharmazeutisch annehmbares Salz von Indacaterol zu erhalten.

5. Verfahren nach einem der vorhergehenden Ansprüche, wobei R² zusammen mit dem benachbarten Sauerstoff ein Acetal, einen Ether, einen Silylether oder einen Ester bildet.

6. Verfahren nach einem der vorhergehenden Ansprüche, wobei R² aus der Gruppe ausgewählt ist, die aus 1-(n-Butoxy)-ethyl und Tetrahydro-pyran-2-yl besteht.

7. Verfahren nach einem der vorhergehenden Ansprüche, wobei die Säure HA aus der Gruppe ausgewählt ist, die aus L-Weinsäure, Dibenzoyl-L-Weinsäure, Mandelsäure, Bernsteinsäure und Benzoesäure besteht.

8. Verfahren nach einem der vorhergehenden Ansprüche, wobei X Chlor oder Brom ist und wobei ein Iodidsalz, wie etwa Natriumiodid, in dem Schritt des Umsetzens der Verbindungen der Formel I und II zugegeben wird.

9. Verfahren nach einem der vorhergehenden Ansprüche, wobei das Endprodukt des Verfahrens das Maleatsalz von Indacaterol ist.

10. Verfahren nach einem der vorhergehenden Ansprüche zur Herstellung eines pharmazeutisch annehmbaren Salzes von Indacaterol, wobei das Verfahren Erhalten von Indacaterol in Form einer freien Base, Isolieren davon in fester Form und Umsetzen davon mit einer geeigneten Säure, wie etwa Maleinsäure, umfasst.

11. Verbindung der Formel I: 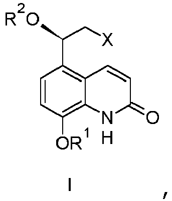 wobei R¹, R² und X wie in einem der Ansprüche 1 bis 10 definiert sind, mit der Maßgabe, dass R² nicht tert-Butyl(dimethyl)silyl oder Tetrahydro-2H-pyran-2-yl ist, wenn R¹ Benzyl ist und X Br ist.

12. Verbindung der Formel III: 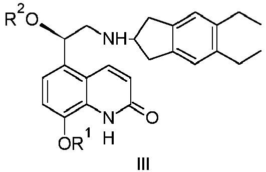 oder ein Salz davon, wobei R¹ und R² wie in einem der Ansprüche 1 bis 10 definiert sind.

## Revendications

1. Procédé pour la préparation d'Indacatérol ou d'un sel de celui-ci de qualité pharmaceutique, comprenant la réaction du composé de formule I avec 2-amino-5,6-diéthylindan de formule II, de préférence en présence d'une base, en le composé de formule III, puis la conversion du composé de formule III en Indacatérol ou en un sel de celui-ci de qualité pharmaceutique : 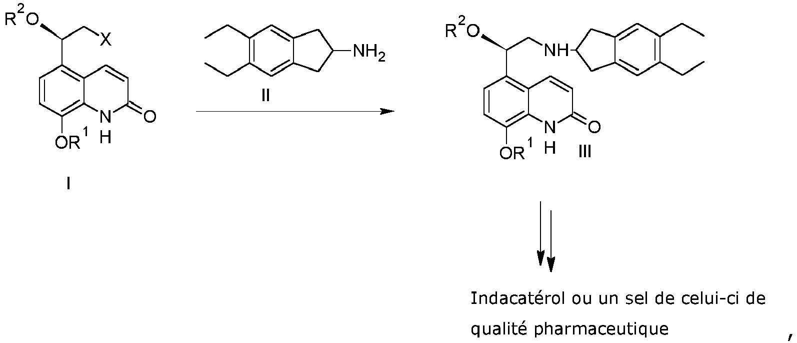 où R¹ est benzyle, R² est un groupe protecteur qui est stable dans des conditions légèrement alcalines, et X est un halogène sélectionné dans le groupe constitué de chloro, bromo et iodo.

2. Procédé selon la revendication 1, dans lequel le composé de formule III est converti en Indacatérol en étant d'abord converti en un composé de formule IV par l'enlèvement préalable du groupe protecteur R² par adjonction d'un acide, de préférence un acide aqueux, suivie de l'adjonction de l'acide HA : 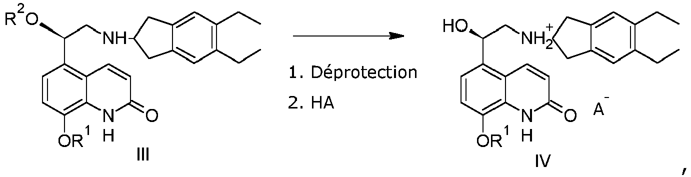 puis la conversion du composé de formule IV en Indacatérol ou en un sel de celui-ci de qualité pharmaceutique.

3. Procédé selon la revendication 2, comprenant en outre la précipitation d'un sel acide d'Indacatérol protégé de formule IV en présence d'eau et d'un solvant organique miscible dans l'eau, puis la conversion du sel acide d'Indacatérol protégé précipité de formule IV en Indacatérol ou en un sel de celui-ci de qualité pharmaceutique :

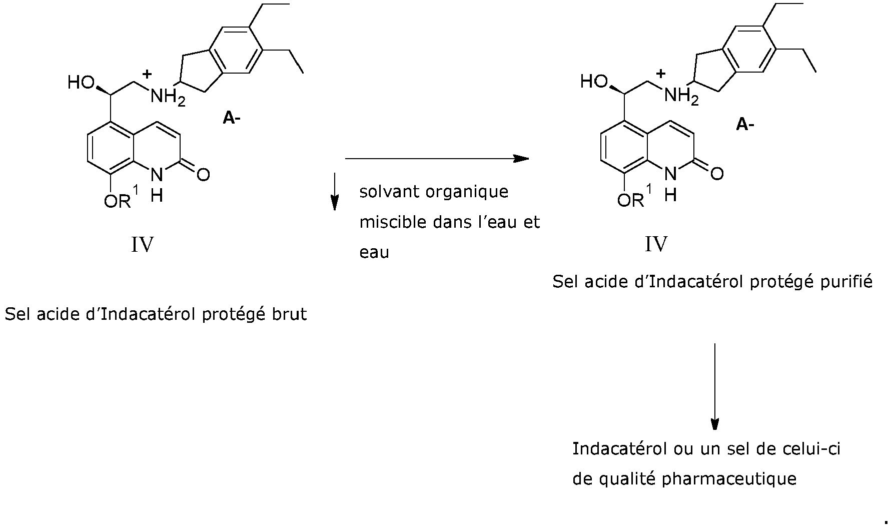

4. Procédé selon une revendication 1 à 3, dans lequel le composé de formule IV est converti en Indacatérol ou en un sel de celui-ci de qualité pharmaceutique par :
a) neutralisation du composé de formule IV, enlèvement du benzyle pour obtenir une base libre d'Indacatérol en solution ou en suspension, isolation optionnelle de la base libre d'Indacatérol sous forme solide et, optionnellement, obtention d'un sel d'Indacatérol de qualité pharmaceutique par adjonction d'un acide approprié, tel que l'acide maléique, à la base libre ;
b) enlèvement du benzyle pour obtenir un composé de formule V : 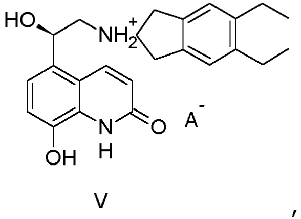 neutralisation du composé de formule V pour obtenir la base d'Indacatérol libre, en solution ou en suspension, isolation optionnelle de la base libre d'Indacatérol sous forme solide et, optionnellement, obtention d'un sel d'Indacatérol de qualité pharmaceutique par adjonction d'un acide approprié, tel que l'acide maléique, à la base libre ; ou
c) enlèvement du benzyle pour obtenir un composé de formule V, réaction du composé de formule V directement avec un acide approprié, tel que l'acide maléique, pour obtenir un sel d'Indacatérol de qualité pharmaceutique.

5. Procédé selon l'une quelconque des revendications précédentes, dans lequel R² forme un acétal, un éther, un éther de silyle, ou un ester conjointement avec l'oxygène adjacent.

6. Procédé selon l'une quelconque des revendications précédentes, dans lequel R² est sélectionné dans le groupe constitué de 1-(n-butoxy)-éthyle et tétrahydro-pyran-2-yle.

7. Procédé selon l'une quelconque des revendications précédentes, dans lequel l'acide HA est sélectionné dans le groupe constitué de : acide L-tartrique, acide dibenzoyl-L-tartrique, acide mandélique, acide succinique et acide benzoïque.

8. Procédé selon l'une quelconque des revendications précédentes, dans lequel X est chloro ou bromo, et dans lequel un sel iodure, tel qu'iodure de sodium, est ajouté dans l'étape de réaction des composés de formules I et II.

9. Procédé selon l'une quelconque des revendications précédentes, dans lequel le produit final du procédé est le sel maléate d'Indacatérol.

10. Procédé selon l'une quelconque des revendications précédentes pour la préparation d'un sel d'Indacatérol de qualité pharmaceutique, ledit procédé comprenant l'obtention d'une base libre d'Indacatérol, son isolation sous forme solide, et sa mise en réaction avec un acide approprié, tel que l'acide maléique.

11. Composé de formule I : 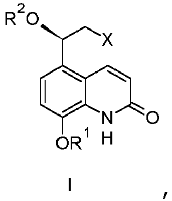 dans lequel R¹, R² et X sont tels que définis dans l'une quelconque des revendications 1 à 10, sous réserve que, quand R¹ est benzyle et que X est Br, R² n'est pas tert-butyl(diméthyl)silyle ni tétrahydro-2H-pyran-2-yle.

12. Composé de formule III : 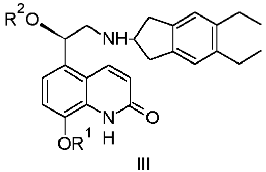 ou un sel de celui-ci, dans lequel R¹ et R² sont tels que définis dans l'une quelconque des revendications 1 à 10.
